# EUROPÄISCHE PATENTSCHRIFT

(11) **EP 2 212 688 B1**
(45) Veröffentlichungstag und Bekanntmachung des Hinweises auf die Patenterteilung: **15.06.2011**
(21) Anmeldenummer: 08850527.6
(22) Anmeldetag: 10.11.2008
(51) Int. Cl.: G01N 33/28, G01N 27/22

(54) **VERFAHREN ZUR BESTIMMUNG EINES KRAFTSTOFFANTEILS IN EINEM MOTORÖL EINES KRAFTFAHRZEUGS**
METHOD FOR DETERMINING A FUEL PORTION IN A MOTOR OIL OF A MOTOR VEHICLE
PROCÉDÉ POUR DÉTERMINER LA TENEUR EN CARBURANT D'UNE HUILE MOTEUR D'UN VÉHICULE AUTOMOBILE

(30) Priorität: 16.11.2007 DE 102007054858
(43) Veröffentlichungstag der Anmeldung: 04.08.2010
(73) Patentinhaber: Continental Automotive GmbH, 30165 Hannover (DE); Continental Automotive France, 31100 Toulouse (FR)
(72) Erfinder: BIERL, Rudolf, 93055 Regensburg (DE); GRASS, Philippe, 93053 Regensburg (DE); HAAG, Jan, 93149 Nittenau (DE); HOLLSTEIN, Armin, 93057 Regensburg (DE); SCHÄDLICH, Denny, 08223 Neustadt (DE)
(86) Internationale Anmeldenummer: PCT/EP2008/065234
(87) Internationale Veröffentlichungsnummer: WO 2009/062913

(56) Entgegenhaltungen:
- WO-A-03/014729
- US-A- 4 646 070
- US-A1- 2002 011 095
- US-A1- 2003 222 656
- US-A1- 2006 229 776

## Beschreibung

Die vorliegende Erfindung betrifft ein Verfahren zur Bestimmung eines Kraftstoffanteils in einem Motoröl eines Kraftfahrzeugs. Im Betrieb von Verbrennungskraftmaschinen von Kraftfahrzeugen kann Kraftstoff in das Motoröl eindringen. Diese Gefahr besteht insbesondere bevor der Motor seine Betriebstemperatur erreicht hat, also im kalten Zustand. So erfolgt vor Erreichen der Betriebstemperatur der Verbrennungskraftmaschine oftmals keine vollständige Verbrennung des zugeführten Kraftstoffs. Der Kraftstoff kann sich dann beispielsweise an den Zylinderwänden niederschlagen und in das Motoröl eindringen. Außerdem bestehen im kalten Zustand des Motors Luftspalte zwischen Motorkomponenten, durch die der nicht verbrannte Kraftstoff in das Motoröl eindringen kann. Aufgrund ihrer nicht flüchtigen Bestandteile besteht dieses Problem insbesondere bei Dieselkraftstoffen. Dabei kommt es besonders häufig zu einem Eindringen von Kraftstoff in das Motoröl bei so genannten Bio-Dieselkraftstoffen, also Dieselkraftstoffen auf pflanzlicher Basis, die beispielsweise aus Pflanzenölen oder tierischen Fetten gewonnen werden, da diese auch bei Erreichen der Betriebstemperatur der Verbrennungskraftmaschine nicht immer vollständig verbrennen.

Das Motoröl erfüllt in einer Verbrennungskraftmaschine eine Vielzahl wichtiger Aufgaben. Es dient vorrangig zur Schmierung der relativ gegeneinander bewegten Teile in Verbrennungskraftmaschinen. Darüber hinaus werden von dem Motoröl Reibungswärme abgeführt, Verunreinigungen ausgewaschen und Metallteile vor Korrosion geschützt. Durch den in das Motoröl eindringenden Kraftstoff steigt der Motorverschleiß stark an, oftmals begleitet von einer Zunahme des Kraftstoffverbrauchs und der Schadstoffemissionen der Verbrennungskraftmaschine. Zur Lösung dieses Problems wurden bislang die Motorölwechselintervalle, beispielsweise bei Einsatz von Bio-Kraftstoffen, entsprechend verkürzt. Dabei ist jedoch vor dem Ölwechsel keine Aussage darüber möglich, ob der Ölwechsel zu diesem Zeitpunkt tatsächlich aufgrund eines erhöhten Kraftstoffanteils im Motoröl erforderlich ist. Somit werden unter Umständen unnötige Ölwechsel durchgeführt.

Die US 2006/229776 A1 offenbart eine Vorrichtung zur Bestimmung eines Kraftstoffanteils in einem Motoröl eines Kraftfahrzeuges mit mindestens zwei, einen Kondensator bildenden Elektroden, zwischen die das Motoröl gebracht werden kann. An die Elektroden wird ein oszillierendes Signal mit einer hohen und einer niedrigen Frequenz angelegt, wobei das Fluid auf diese Signale reagiert.

Ausgehend von dem voran erläuterten Stand der Technik liegt der Erfindung daher die Aufgabe zugrunde, ein Verfahren der eingangs genannten Art anzugeben, mit denen der Kraftstoffanteil im Motoröl in einfacher Weise bestimmt werden kann, so dass rechtzeitig ein zu hoher Kraftstoffanteil im Motoröl festgestellt werden kann.

Diese Aufgabe wird erfindungsgemäß durch den Gegenstand des unabhängigen Anspruchs 1 gelöst. Vorteilhafte Ausgestaltungen und Weiterbildungen der Erfindung finden sich in den abhängigen Patentansprüchen sowie der Beschreibung und den Figuren.

Gemäß der Erfindung wird die Aufgabe durch ein Verfahren zur Bestimmung eines Kraftstoffanteils in einem Motoröl eines Kraftfahrzeugs gelöst, bei dem das Motoröl zwischen mindestens zwei einen Kondensator bildende Elektroden gebracht wird, bei dem die Kapazität des Kondensators bestimmt wird während sich das Motoröl zwischen den Elektroden befindet, und bei dem aus der Kapazität des Kondensators der Kraftstoffanteil im Motoröl bestimmt wird. Wechselspannungen unterschiedlicher Frequenzen werden an die Kondensatorelektroden angelegt und die Impedanz des Kondensators wird jeweils bestimmt. Aus den Impedanzwerten bei unterschiedlichen Wechselspannungsfrequenzen wird die Art des in dem Motoröl vorhandenen Kraftstoffs identifiziert.

Der Erfindung liegt die Idee zugrunde, den Kraftstoffanteil im Motoröl kapazitiv zu messen. Dabei basiert die Erfindung auf der Erkenntnis, dass unterschiedliche Kraftstoff- und Ölsorten verschiedene Dielektrizitätszahlen besitzen. Die Dielektrizitätszahl des Motoröls verändert sich daher mit einem zunehmenden Kraftstoffanteil im Öl. Zur Bestimmung der Dielektrizitätszahl wird das zu messende Motoröl in eine Messzelle in dem Sensor eingebracht, die einen Kondensator bildet. Eine Änderung der Dielektrizitätszahl des zwischen den Kondensatorelektroden befindlichen Motoröls führt entsprechend zu einer Änderung des Dielektrikums des Kondensators und damit einer Änderung der Kapazität des Kondensators. Durch eine Messung der Kapazität des Kondensators während sich das Motoröl zwischen den Kondensatorelektroden befindet, kann also in genauer Weise auf den im Motoröl befindlichen Kraftstoffanteil geschlossen werden. Insbesondere ist eine kritische Kraftstoffverunreinigung des Motoröls mit dem erfindungsgemäßen Verfahren in zuverlässiger und präziser Weise feststellbar. Bei Überschreiten einer maximalen Kraftstoffverunreinigung, einem Grenzwert, können dann geeignete Maßnahmen ergriffen werden. Dazu zählen beispielsweise entsprechende Informationen an den Kraftfahrzeugführer oder eine Werkstatt, beispielsweise im Rahmen einer Inspektion des Fahrzeugs.

Der Aufbau eines für die Erfindung verwendbaren Sensors zur Messung eines Kraftstoffanteils in einem Motoröl ist dabei besonders einfach und daher kostengünstig. Entsprechend kann mit dem erfindungsgemäßen Verfahren der Kraftstoffanteil im Motoröl in besonders einfacher und kostengünstiger Weise ermittelt werden.

Das Motoröl wird erfindungsgemäß zwischen die Elektroden gebracht. Dabei kann es zu einem späteren Zeitpunkt wieder aus dem Raum zwischen den Elektroden entfernt werden. Das Motoröl kann zwischen den Elektroden hindurch geleitet werden. Durchleiten des Öls zwischen den Elektroden bedeutet in diesem Zusammenhang, dass das Öl zur Messung zwischen die Elektroden gebracht wird und zu einem späteren Zeitpunkt wieder aus dem Sensor austritt.

Das Motoröl kann in besonders einfacher Weise im Rahmen des normalen Ölkreislaufs zwischen die Kondensatorelektroden gebracht werden. Dazu kann der Sensor in geeigneter Weise in den Ölkreislauf eingebracht werden, insbesondere in diesem angeordnet sein. In diesem Fall fließt das Öl also im Rahmen seines normalen Kreislaufs zwischen den Elektroden hindurch. Selbstverständlich ist es grundsätzlich auch denkbar, eine separate Einrichtung zum Einbringen des Motoröls zwischen die Elektroden vorzusehen, beispielsweise eine geeignete Pumpe.

Es ist auch denkbar, den Sensor zur Messung in das Motoröl einzutauchen und das Öl auf diese Weise zwischen die Elektroden zu bringen. Der Sensor kann beispielsweise auch in einer Ölwanne des Verbrennungsmotors angeordnet werden und das Motoröl kann auf diese Weise zwischen die Elektroden gebracht werden. In diesem Fall ist der Sensor also nicht in ein Kreislaufsystem eingebunden. Der Ein- und Austritt des Motoröls in den Sensor und insbesondere zwischen die Elektroden erfolgt dann durch Konvektion in der Ölwanne.

Es ist möglich, die im Betrieb gemessene Kapazität des Kondensators mit einer im Rahmen einer vorhergehenden Kalibrierung gemessenen Kapazität bei reinem, also nicht mit Kraftstoff verunreinigtem Motoröl zu vergleichen. Auf dieser Grundlage kann in besonders einfacher Weise der Grad der Kraftstoffverunreinigung festgestellt werden.

Zur Bestimmung der Kapazität des Kondensators kann in an sich bekannter Weise eine elektrische Spannung, insbesondere eine Wechselspannung an die Kondensatorelektroden angelegt werden. Dazu kann die Vorrichtung eine geeignete Spannungsversorgung aufweisen. Die Kapazitätsbestimmung von Kondensatoren ist dem Fachmann an sich bekannt und soll daher nicht näher erläutert werden.

In dem vorliegenden Zusammenhang ist von dem Begriff Bestimmung der Kapazität selbstverständlich auch die Bestimmung einer mit der Kapazität des Kondensators korrelierenden Größe umfasst. Selbstverständlich kann ebenfalls aus einer solchen mit der Kapazität korrelierenden Größe des Kondensators der Kraftstoffanteil im Öl bestimmt werden.

Gemäß einer Ausgestaltung der Erfindung kann es sich bei dem Kraftstoffanteil um einen Dieselkraftstoffanteil handeln. Die Verbrennungskraftmaschine kann also eine Dieselverbrennungskraftmaschine sein. Aufgrund ihrer nicht flüchtigen Bestandteile besteht insbesondere bei Dieselkraftstoffen das Problem des Eindringens von Kraftstoff in das Motoröl, so dass ein hoher Bedarf besteht, den Kraftstoffanteil im Motoröl zu kennen.

Es ist selbstverständlich auch denkbar, dass es sich bei dem Kraftstoffanteil im Motoröl um einen Benzinkraftstoffanteil, also einen Kraftstoff für einen Otto-Verbrennungsmotor handelt. In diesem Fall kann es sich bei der Verbrennungskraftmaschine um eine Otto-Verbrennungskraftmaschine handeln.

Gemäß einer weiteren Ausgestaltung kann es sich bei dem Kraftstoffanteil um einen Bio-Kraftstoffanteil handeln, insbesondere einen Bio-Dieselkraftstoffanteil. Derartige Kraftstoffe auf pflanzlicher Basis verbrennen teilweise auch bei Betriebstemperatur der Brennkraftmaschine nicht vollständig. Dadurch kann es zu einem Eindringen des Kraftstoffs in das Motoröl kommen. Entsprechend ist es besonders wichtig, den Kraftstoffanteil im Öl zu überwachen. Darüber hinaus ist ein Bio-Kraftstoff, insbesondere ein Bio-Dieselkraftstoff im Motoröl mittels einer Kapazitätsmessung besonders gut bestimmbar, da sich die Dielektrizitätszahlen von Bio-Kraftstoff besonders deutlich von den Dielektrizitätszahlen von Motorölen unterscheiden. Insbesondere ist dieser Unterschied größer als beispielsweise zwischen mineralischem Dieselkraftstoff und mineralischem Motoröl.

Gemäß der Erfindung werden Wechselspannungen unterschiedlicher Frequenzen an die Kondensatorelektroden angelegt und jeweils die Impedanz des Kondensators bestimmt. Es kann eine Spannungsversorgung vorgesehen sein, mit der Wechselspannungen unterschiedlicher Frequenzen an die Kondensatorelektroden anlegbar sind, wobei mit einer Auswerteeinrichtung jeweils die Impedanz des Kondensators bestimmbar ist. Die elektrischen Wechselspannungen werden dabei angelegt während sich das Motoröl zwischen den Elektroden befindet. Die Impedanz ist frequenzabhängig. Das Bestimmen der Impedanz ist dem Fachmann an sich bekannt und wird daher nicht näher erläutert. Gemäß dieser Ausgestaltung wird also ein Wechselspannungsfrequenzbereich durchfahren und ein entsprechendes Impedanzspektrum aufgenommen. Daraus lassen sich besonders genaue Informationen über den Kraftstoffanteil im Motoröl gewinnen. Insbesondere ist es dann möglich, aus den Impedanzwerten bei unterschiedlichen Wechselspannungsfrequenzen die Art des in dem Motoröl vorhandenen Kraftstoffs zu identifizieren. Diese Identifizierung des Kraftstoffs kann mit der Auswerteeinrichtung erfolgen. Die Erfindung beruht auf der Erkenntnis, dass sich unterschiedliche Kraftstoffe bei unterschiedlichen Wechselspannungsfrequenzen in unterschiedlicher Weise auf die Impedanz auswirken. Aus einer Auswertung des aufgenommenen Impedanzspektrums, beispielsweise der Lage und/oder der Höhe von Maxima (Peaks) und Minima usw., lassen sich unterschiedliche Kraftstoffe identifizieren. Es lässt sich auf diese Weise beispielsweise feststellen, ob es sich bei dem in dem Motoröl vorhandenen Kraftstoff um einen mineralischen oder einen Bio-Dieselkraftstoff handelt. Zur Auswertung kann das aufgenommene Impedanzspektrum mit zuvor bei bekannten Kraftstoffverunreinigungen erstellten Referenzspektren verglichen werden.

Gemäß einer besonders praxisgemäßen Ausgestaltung kann das Motoröl zwischen eine Mehrzahl einen Kondensator bildende Elektroden gebracht werden. Bei dieser Ausgestaltung kann eine Mehrzahl einen Kondensator bildenden Elektroden vorgesehen sein. Durch eine Mehrzahl an Kondensatorelektroden lässt sich ein Kondensator mit einer besonders großen Kapazität pro Volumen bilden. Dadurch stehen betragsmäßig entsprechend hohe Kapazitätsmesswerte zur Verfügung. Beispielsweise induzierte Fremdfelder können nur geringen Einfluss auf die Messungen nehmen. Dadurch können besondere und entsprechend teure Abschirmmaßnahmen entfallen. Wenn mehrere Elektroden vorgesehen sind, können diese insbesondere jeweils doppelseitig wirken. Dazu wird jeweils zwischen benachbarten Elektroden eine elektrische Spannung angelegt. Benachbarte Elektroden liegen also an einem unterschiedlichen elektrischen Potential. Dabei kann jeweils jede zweite Elektrode an demselben Potential liegen. Auf diese Weise ist nur eine Spannungsversorgung mit zwei elektrischen Anschlüssen für sämtliche Kondensatorelektroden erforderlich.

Die Kondensatorelektroden können Kondensatorplatten sein. Die Ausbildung der Elektroden in Plattenform führt zu einem besonders einfachen Aufbau des Kondensators. Die Platten sind dann üblicherweise parallel zueinander angeordnet und besitzen einen Abstand zueinander, in dem sich das Dielektrikum des Kondensators befindet, wobei benachbarte Platten an einem unterschiedlichen elektrischen Potential liegen.

Insbesondere wenn eine Mehrzahl von Kondensatorplatten vorgesehen ist, zwischen die das Motoröl zur Messung gebracht wird, können diese Kondensatorplatten parallel und beabstandet zueinander angeordnet sein. Die Platten können also mit einem Abstand zueinander gestapelt angeordnet sein. Dabei liegen üblicherweise wieder benachbarte Elektroden an einem unterschiedlichen elektrischen Potential. Diese an einem unterschiedlichen Potential liegenden Elektroden können dabei kammförmig ineinander greifen. Jeweils jede zweite Platte liegt dann an demselben Potential. Wiederum ergibt sich auf diese Weise ein besonders einfacher Aufbau, wobei nur eine Spannungsversorgung mit zwei elektrischen Anschlüssen für sämtliche Platten erforderlich ist. Natürlich können die Elektroden auch eine andere Form besitzen, beispielsweise zylindrisch ausgebildet sein. Dann können diese zylindrischen Elektroden einen unterschiedlichen Durchmesser aufweisen und ineinander geschoben angeordnet sein. Zwischen benachbarten Zylindern kann dann jeweils eine elektrische Spannung angelegt werden.

Ein Ausführungsbeispiel der Erfindung wird nachfolgend anhand einer Zeichnung näher erläutert. Es zeigen schematisch:
- Fig. 1: eine für die Erfindung verwendbare Vorrichtung in einer Seitenan- sicht, und
- Fig. 2: die in Fig. 1 dargestellte Vorrichtung in einer Schnittansicht.

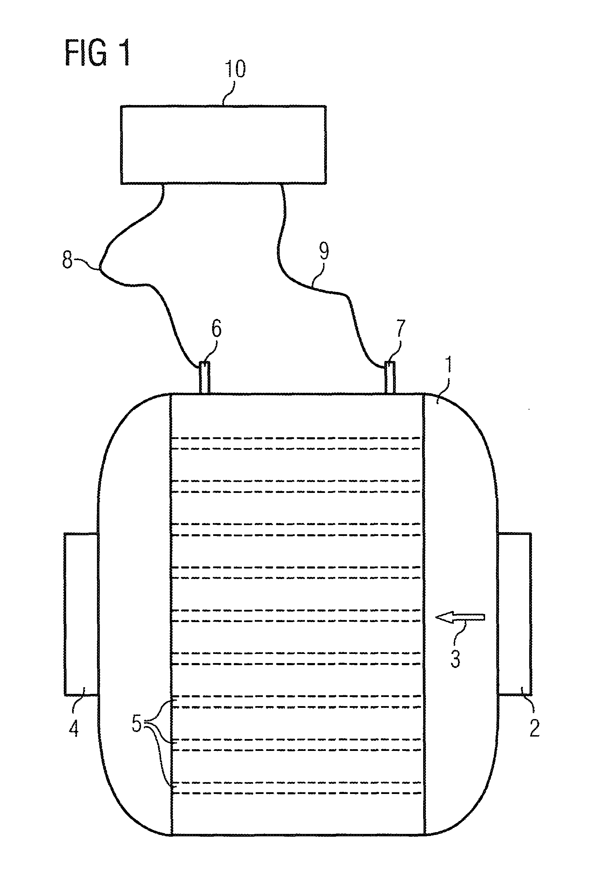

In den Figuren bezeichnen gleiche Bezugszeichen gleiche Gegenstände. In Fig. 1 ist eine Vorrichtung zur Messung eines Kraftstoffanteils in einem Motoröl eines Kraftfahrzeugs in einer Seitenansicht dargestellt. Die Vorrichtung besitzt ein Gehäuse 1, im vorliegenden Beispiel aus Kunststoff. An dem Gehäuse 1 ist eine Eintrittsöffnung 2 für das zu untersuchende Motoröl vorgesehen. Zur Messung kann das Motoröl durch die Eintrittsöffnung 2 hindurch in die Vorrichtung geleitet werden. Die Vorrichtung ist in dem dargestellten Beispiel in den Ölkreislauf des Motors integriert. Das Motoröl fließt dann durch die Vorrichtung hindurch, wie durch den Pfeil 3 in Fig. 1 veranschaulicht. Nach Durchlaufen der Vorrichtung tritt das Motoröl an einer der Eintrittsöffnung 2 gegenüberliegenden Austrittsöffnung 4 wieder aus der Vorrichtung heraus. Bei dem durch die Vorrichtung geleiteten Motoröl handelt es sich in dem dargestellten Beispiel um ein Motoröl einer Dieselverbrennungskraftmaschine. In dem dargestellten Beispiel ist dieses durch einen Bio-Dieselkraftstoffanteil verunreinigt.

In dem dargestellten Beispiel besitzt die Vorrichtung eine Mehrzahl plattenförmiger Elektroden, die einen Kondensator bilden. In Fig. 1 sind lediglich einiger dieser hinter der Wand des Gehäuses 1 liegenden Elektroden 5 schematisch angedeutet. Das Motoröl fließt entsprechend in Richtung des Pfeils 3 zwischen diesen einen Kondensator bildenden Elektroden 5 hindurch. Zur Versorgung der Kondensatorplatten mit einer elektrischen Spannung ist eine gemeinsame Spannungsversorgung vorgesehen. Diese weist in dem dargestellten Beispiel zwei elektrische Anschlüsse 6, 7 auf. Über elektrische Leitungen 8, 9 sind die elektrischen Anschlüsse jeweils mit einer Auswerteeinrichtung 10 verbunden, in die vorliegend auch die Spannungsversorgung integriert ist.

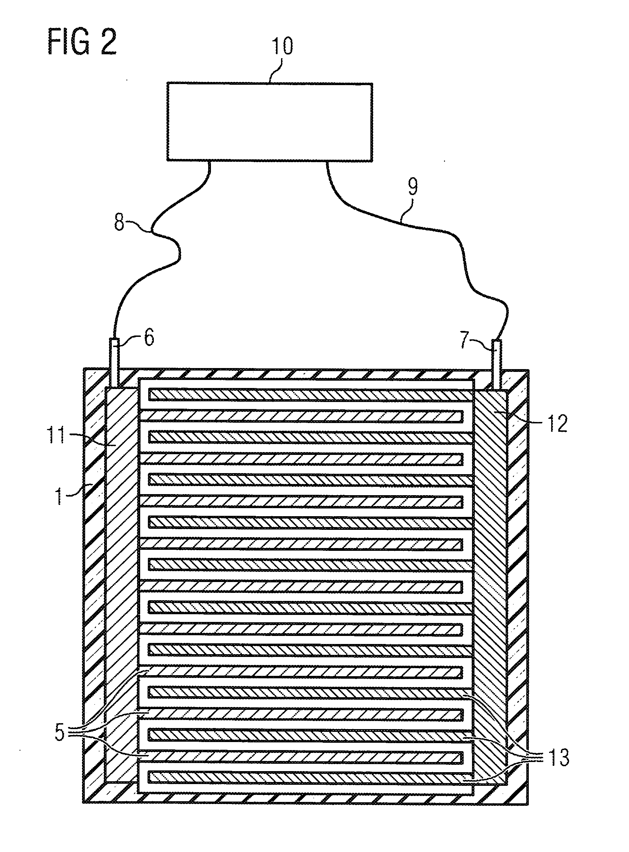

Der Aufbau der Kondensatorplatten der Vorrichtung ist in Fig. 2 zu erkennen. In Fig. 2 ist die Vorrichtung in einer senkrecht zur Fließrichtung 3 des Motoröls geschnittenen Ansicht dargestellt. Das Motoröl fließt in der Darstellung in Fig. 2 also senkrecht zu der Zeichenebene durch die Vorrichtung hindurch. Die elektrischen Anschlüsse 6, 7 sind jeweils mit einer ersten Anschlussplatte 11 und einer zweiten Anschlussplatte 12 des Kondensators verbunden. Von der ersten Anschlussplatte 11 geht dabei rechtwinklig eine Mehrzahl von ersten Kondensatorplatten 5 ab. Von der zweiten Anschlussplatte 12 gehen entsprechend ebenfalls rechtwinklig eine Mehrzahl zweiter Kondensatorplatten 13 ab. Die ersten und zweiten Kondensatorplatten 5, 13 sind übereinander parallel und beabstandet zueinander angeordnet. Bei dieser gefiederten Anordnung der Kondensatorplatten greifen die ersten Platten 5 kammartig in die zweiten Platten 13 ein. Es sind also abwechselnd erste Kondensatorplatten 5 und zweite Kondensatorplatten 13 benachbart angeordnet.

Die ersten Kondensatorplatten 5 liegen über die erste Anschlussplatte 11 und den ersten elektrischen Anschluss 6 an demselben gemeinsamen elektrischen Potential. Die zweiten Kondensatorplatten 13 liegen dagegen über die zweite Anschlussplatte 12 und den zweiten elektrischen Anschluss 7 an einem gemeinsamen, von dem elektrischen Potential der ersten Kondensatorplatten 5 unterschiedlichen elektrischen Potential. Auf diese Weise ist gewährleistet, dass jeweils benachbarte Kondensatorplatten 5, 13 an einem unterschiedlichen elektrischen Potential liegen, zwischen diesen Platten also jeweils eine elektrische Spannung angelegt ist. Bis auf die unterste und die oberste Kondensatorplatte in Fig. 2 wirken die Platten also doppelseitig. Der Kondensator besitzt daher eine verhältnismäßig große Kapazität pro Volumen. Dadurch wirken sich Fremdeinflüsse, beispielsweise induzierte Fremdfelder, nur wenig auf die Messung aus. Auf aufwendige Abschirmmaßnahmen kann daher verzichtet werden.

Im Betrieb der Vorrichtung wird das Motoröl in Fließrichtung 3 durch die Vorrichtung hindurchgeleitet, wobei das Öl zwischen den Kondensatorplatten 5, 13 hindurchfließt. Während sich das Motoröl zwischen den Kondensatorplatten 5, 13 befindet, wird mittels der Auswerteeinrichtung 10 die Kapazität des durch die Platten 5, 13 gebildeten Kondensators bestimmt. Mit verändertem Kraftstoffanteil in dem Motoröl verändert sich die Dielektrizitätszahl des Motoröls und damit die gemessene Kapazität. Aus der gemessenen Kapazität wird wiederum mittels der Auswerteeinrichtung beispielsweise anhand im Rahmen einer vorhergehenden Kalibrierung ermittelter Kennfelder der Kraftstoffanteil in dem Motoröl bestimmt.

Um die Art des in dem Motoröl vorhandenen Kraftstoffs zu identifizieren, werden mittels der Auswerteeinrichtung 10 Wechselspannungen unterschiedlicher Frequenzen an die Kondensatorplatten 5, 13 angelegt. Insbesondere wird dabei ein Wechselspannungsfrequenzbereich durchfahren. Ebenfalls mittels der Auswerteeinrichtung 10 wird jeweils zu den verschiedenen Wechselspannungsfrequenzen die Impedanz des Kondensators bestimmt. Es wird also ein Impedanzspektrum aufgenommen. Anhand des Impedanzspektrums wird dann von der Auswerteeinrichtung 10 die Kraftstoffart identifiziert, die sich in dem Motoröl befindet. Dazu wertet die Auswerteeinrichtung 10 unter anderem die Lage und Höhe von Maxima und Minima des Impedanzspektrums aus. Die Auswertung erfolgt in dem dargestellten Beispiel durch einen Vergleich mit zuvor im Rahmen einer Kalibrierung erstellen Impedanzspektren bei bekannten Kraftstoffverunreinigungen.

Mit dem erfindungsgemäßen Verfahren kann in einfacher und kostengünstiger Weise der Kraftstoffanteil in dem Motoröl zuverlässig ermittelt werden.

## Patentansprüche

1. Verfahren zur Bestimmung eines Kraftstoffanteils in einem Motoröl eines Kraftfahrzeugs,
- bei dem das Motoröl zwischen mindestens zwei einen Kondensator bildende Elektroden (5, 13) gebracht wird,
- bei dem die Kapazität des Kondensators bestimmt wird während sich das Motoröl zwischen den Elektroden (5, 13) befindet, und
- bei dem aus der Kapazität des Kondensators der Kraftstoffanteil im Motoröl bestimmt wird, wobei
- Wechselspannungen unterschiedlicher Frequenzen an die Kondensatorelektroden (5, 13) angelegt werden und jeweils die Impedanz des Kondensators bestimmt wird, **dadurch gekennzeichnet, dass**
- aus den Impedanzwerten bei unterschiedlichen Wechselspannungsfrequenzen die Art des in dem Motoröl vorhandenen Kraftstoffs identifiziert wird.

2. Verfahren nach Anspruch 1, **dadurch gekennzeich**ne t, dass es sich bei dem Kraftstoffanteil um einen Dieselkraftstoffanteil handelt.

3. Verfahren nach Anspruch 1, **dadurch gekennzeich**ne t, dass es sich bei dem Kraftstoffanteil um einen Benzinkraftstoffanteil handelt.

4. Verfahren nach einem der Ansprüche 1 bis 3, **dadurch gekennzeichnet, dass** es sich bei dem Kraftstoffanteil um einen Bio-Kraftstoffanteil handelt.

5. Verfahren nach einem der vorhergehenden Ansprüche, **dadurch gekennzeichnet, dass** das Motoröl zwischen eine Mehrzahl einen Kondensator bildenden Elektroden (5, 13) gebracht wird.

6. Verfahren nach einem der vorhergehenden Ansprüche, **dadurch gekennzeichnet, dass** die Elektroden (5, 13) Kondensatorplatten (5, 13) sind.

7. Verfahren nach den Ansprüchen 5 und 6, **dadurch gekennzeichnet, dass** die Kondensatorplatten (5, 13) parallel und beabstandet zueinander angeordnet sind.

## Claims

1. Method for determining a fuel portion in an engine oil of a motor vehicle,
- in which the engine oil is placed between at least two electrodes (5, 13) which form a capacitor,
- in which the capacitance of the capacitor is determined while the engine oil is located between the electrodes (5, 13), and
- in which the fuel portion in the engine oil is determined from the capacitance of the capacitor,
- alternating voltages of different frequencies are applied to the capacitor electrodes (5, 13) and the impedance of the capacitor is determined in each case, **characterized in that**
- the type of fuel present in the engine oil is identified from the impedance values at different alternating voltage frequencies.

2. Method according to Claim 1, **characterized in that** the fuel portion is a diesel fuel portion.

3. Method according to Claim 1, **characterized in that** the fuel portion is a gasoline fuel portion.

4. Method according to one of Claims 1 to 3, **characterized in that** the fuel portion is a bio-fuel portion.

5. Method according to one of the preceding claims, **characterized in that** the engine oil is placed between a plurality of electrodes (5, 13) which form a capacitor.

6. Method according to one of the preceding claims, **characterized in that** the electrodes (5, 13) are capacitor plates (5, 13).

7. Method according to Claims 5 and 6, **characterized in that** the capacitor plates (5, 13) are arranged in parallel and spaced apart from one another.

## Revendications

1. Procédé pour déterminer une teneur de carburant d'une huile de moteur d'un véhicule à moteur,
- dans lequel on met l'huile de moteur entre au moins deux électrodes qui forment un condensateur (5, 13),
- dans lequel on détermine la capacité du condensateur pendant que l'huile de moteur se trouve entre les électrodes (5, 13), et
- dans lequel sur la base de la capacité du condensateur, on détermine la teneur en carburant de l'huile de moteur,
- dans lequel on applique des tensions alternatives de fréquence différentes aux électrodes (5, 13) de condensateur et on détermine l'impédance du condensateur pour chacune d'entre elles,
**caractérisé en ce que**,
- sur la base des valeurs d'impédance pour des fréquences de tension alternatives différentes, on identifie le type de carburant présent dans l'huile de moteur.

2. Procédé suivant la revendication 1, **caractérisé en ce que** la teneur en carburant est une teneur en carburant diesel.

3. Procédé suivant la revendication 1, **caractérisé en ce que** la teneur en carburant est une teneur en carburant d'essence.

4. Procédé suivant l'une des revendications 1 à 3, **caractérisé en ce que** la teneur en carburant est une teneur en biocarburant.

5. Procédé suivant l'une des revendications précédentes, **caractérisé en ce qu'**on met l'huile de moteur entre une pluralité d'électrodes (5, 13) formant un condensateur.

6. Procédé suivant l'une des revendications précédentes, **caractérisé en ce que** les électrodes (5, 13) sont des plateaux (5, 13) de condensateur.

7. Procédé suivant l'une des revendications 5 et 6, **caractérisé en ce que** les plateaux (5, 13) de condensateur sont agencés parallèlement l'un à l'autre et à distance l'un de l'autre.
